(19) **Europäisches Patentamt / European Patent Office / Office européen des brevets**

(11) **EP 3 967 767 A1**

(12) # EUROPEAN PATENT APPLICATION
published in accordance with Art. 153(4) EPC

(43) Date of publication:
**16.03.2022 Bulletin 2022/11**

(51) Int Cl.:
***C12Q 1/68*** (2018.01)

(21) Application number: **20798831.2**

(22) Date of filing: **14.04.2020**

(86) International application number:
**PCT/CN2020/084687**

(87) International publication number:
**WO 2020/220994 (05.11.2020 Gazette 2020/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2019 CN 201910392316**

(71) Applicant: **Mirxes Lab Pte. Ltd.**
**Hangzhou, Zhejiang 310018 (CN)**

(72) Inventor: **ZOU, Ruiyang**
**Hangzhou, Zhejiang 310018 (CN)**

(74) Representative: **Mewburn Ellis LLP**
**Aurora Building**
**Counterslip**
**Bristol BS1 6BX (GB)**

(54) **MICRORNA MARKER COMBINATION FOR DIAGNOSING GASTRIC CANCER AND DIAGNOSTIC KIT**

(57) The present invention relates to the field of molecular biology. Disclosed are a miRNA marker combination and a kit for diagnosing gastric cancer. The miRNA marker combination for diagnosing gastric cancer includes at least four markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p. The marker combination for diagnosing gastric cancer can be used to distinguish blood serum of a patient with gastric cancer from that of a Healthy human. The diagnostic kit of the present invention can diagnosis gastric cancers easily, effectively and non-invasively.

EP 3 967 767 A1

EP 3 967 767 A1

## Description

This application claims the priority of a Chinese patent application with an application number 201910392316.2 filed with the Chinese Patent Office on April 30, 2019 and titled as "miRNA marker combination and kit for diagnosing gastric cancer", the entire content of which is incorporated in this application by reference.

## Technical Field

The invention is in the field of molecular biology, and specifically relates to a miRNA marker combination and a kit for diagnosing gastric cancer.

## Background

Gastric cancer is one of the most common malignant tumors in the world and one of the malignant tumors with the highest mortality rate. Most patients with gastric cancer have missed the best timing of diagnosis and treatment when they are diagnosed, resulting in disease progression, tumor metastasis, and even progressing into the end-stage. From the perspective of TMN staging of gastric cancer, the 5-year survival rate of gastric cancer is 97.6% in the early stage of stage I, 94.9% in the late stage of stage I, 70.49% in stage II, 56.7% in the early stage of stage III, 31.9% in the late stage of stage III, and 6.5% in stage IV. It can be seen that the diagnosis of early gastric cancer is necessary.

Endoscope (gastroscope) is currently the most advantageous tool for diagnosing gastric cancer. The endoscopic manifestations of early gastric cancer include abnormal mucosal color, disappearance of blood vessels on the mucosal surface, depression of or thickening of the bulge in the mucosal layer, irregular nodules, and abnormal mucosal folds around the ulcer. If necessary, part of the tissue can be cut for biopsy. Detection of protein markers in the blood can be used as a reference for the diagnosis of gastric cancer. The tumor protein markers commonly used for gastric cancer include: carcinoembryonic antigen (CEA), carbohydrate antigen 19-9 (CA19-9), carbohydrate antigen 72-4 (CA72-4) and carbohydrate antigen 50 (CA50) and stomach protease, etc., for example.

Biopsy after endoscopy is the gold standard for gastric cancer detection. However, this method is invasive and can cause discomfort and fear in patients. In addition, asymptomatic patients usually do not undergo endoscopy. Conventional tumor protein markers are often used for gastrointestinal cancer detection, but not recommended for gastric cancer diagnosis due to lack of sensitivity and/or specificity.

At present, the detection of miRNA tumor markers in tissues, serum or plasma is used as a reference for the diagnosis of gastric cancer. miRNA is a class of small non-coding single-stranded RNA molecules with a length of about 19-24 nt. Most miRNAs can inhibit the translation of target genes into proteins through complementary binding with the 3'UTR region of target genes, thereby affecting the growth and development of organisms at the cell, tissue or individual level, and participating in various disease processes. The expression profile of miRNA has obvious tissue specificity, and has a specific expression pattern in different tumors. These characteristics make it possible for miRNA to become a new biological marker and therapeutic target for tumor diagnosis. qPCR is the most commonly used method to detect the expression of known miRNAs, which is fast, simple, and reproducible, can quantitatively analyze the expression of miRNAs in a very sensitive and accurate way, and is the most important method in clinical applications. This provides a reliable technical guarantee for circulating miRNAs as non-invasive diagnostic markers for tumor.

Although existing studies have found many promising serum miRNAs for early diagnosis of gastric cancer, these results are not consistent and cannot be mutually verified. The reason lies in the inconsistent selection, collection, and preservation processes of samples among various studies. The content of biomarkers in peripheral blood samples separated and stored using different methods will vary. The body fluid environment between individuals, genetics and other non-cancer factors will affect the expression of miRNA, and a large number of population samples are needed to eliminate this impact. In addition, the detection of miRNA has certain difficulties. Therefore, serum miRNA biomarkers and biomarker combinations that can ultimately be used for gastric cancer screening have not yet been truly determined.

## Summary

In view of this, the purpose of the present invention is to provide a combination of markers and a kit for diagnosing early gastric cancer, so as to distinguish serum of a patient with gastric cancer from that of a Healthy human, and to distinguish serum of a patient with gastric cancer from that of a patient with gastritis, which can detect gastric cancers easily, effectively and non-invasively.

In order to achieve the purpose of the present invention, the present invention adopts the following technical solutions:
The applicant screens and obtains 12 miRNAs as biomarkers and combinations for gastric cancer detection using RT-qPCR technology, specifically hsa-miR-29c-3p, hsa-miR-424-5p, hsa-miR-103a-3p, hsa- miR-93-5p, hsa-miR-181a-5p,

EP 3 967 767 A1

hsa-miR-21-5p, hsa-miR-140-5p, hsa-miR-30e-5p, hsa-miR-142-5p, hsa-miR- 126-3p, hsa-miR-183-5p, and hsa-miR-340-5p. The miRNA marker combination according to the present invention is used to test 4566 subjects. The results are compared with the Helicobacter pylori test and the pepsinogen test, which shows good agreement with the clinical gold standard of endoscopy (AUC is 0.84), and is significantly better than the two existing biomarkers of pepsinogen I/II ratio and Helicobacter pylori test respectively (AUC is 0.62 and 0.64).

A combination of miRNA markers for diagnosing gastric cancer, which includes at least four miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least five miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least six miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least seven miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least eight miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least nine miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least ten miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination includes at least eleven miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination for diagnosing gastric cancer in a peripheral blood sample includes hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination consists of twelve miRNA markers: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the miRNA marker combination consists of eleven miRNA markers: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the miRNA marker combination consists of ten miRNA markers: hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the miRNA marker combination consists of nine miRNA markers: hsa-miR-424-5p, hsa-miR-103a-3p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the miRNA marker combination consists of eight miRNA markers: hsa-miR-424-5p, hsa-miR-103a-3p, hsa-miR-181a-5p, hsa-miR-21-5p, hsa-miR-140-5p, hsa-miR-30e-5p, hsa-miR-126-3p and hsa-miR-340-5p.

In some embodiments, the miRNA marker combination consists of seven miRNA markers: hsa-miR-424-5p, hsa-miR-103a-3p, hsa-miR-181a-5p, hsa-miR-21-5p, hsa-miR-140-5p, hsa-miR-30e-5p and hsa-miR-340-5p.

In some embodiments, the miRNA marker combination consists of six miRNA markers: hsa-miR-424-5p, hsa-miR-103a-3p, hsa-miR-181a-5p, hsa-miR-21-5p, hsa-miR-140-5p and hsa-miR-340-5p.

In some embodiments, the miRNA marker combination consists of five miRNA markers: hsa-miR-103a-3p, hsa-miR-181a-5p, hsa-miR-21-5p, hsa-miR-140-5p and hsa-miR-340-5p.

In some embodiments, the miRNA marker combination consists of four miRNA markers: hsa-miR-103a-3p, hsa-miR-181a-5p, hsa-miR-21-5p and hsa-miR-340-5p.

The present invention also provides a method for identifying a subject at risk of suffering from gastric cancer, including:

3

a. detecting the expression levels of at least four miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p in the subject's peripheral blood sample,

b. calculating and correcting the risk score of the subject with the non-cancer control sample as a reference, and

c. determining the possibility of the subject suffering from gastric cancer based on the risk score.

In some embodiments, the method for determining miRNA expression levels includes the use of quantitative RT-PCR (for example, using SYBR-Green or Tagman-based chemical methods), chip or sequencing. In other embodiments, the biomarker can be detected by Northern blot, droplet digital PCR, mass spectrometry, electrochemiluminescence, or other methods known in the art.

In some embodiments of the method, a linear regression model is used to calculate the risk score.

In some embodiments of the method, the linear regression model used is logistic regression.

In some embodiments of the method, the linear regression model is as follows:

$$\text{Logistic} = (K_{miRNA1} * Ct_{miRNA1}) + (K_{miRNA2} * Ct_{miRNA2}) + (K_{miRNA3} * Ct_{miRNA3}) + \ \ldots\ldots + \text{Constant}$$

wherein miRNA1, miRNA2, miRNA3... are selected from hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p;

CT is the relative expression level of each miRNA detected by qPCR; and

K is the coefficient of each miRNA marker.

The present invention also provides a kit for diagnosing gastric cancer, which includes a reagent for specifically detecting the miRNA marker combination.

In some embodiments, the reagent in the kit according to the present invention is a detection reagent for qPCR method, a detection reagent for chip method or a detection reagent for sequencing method.

In some embodiments, the reagent for specifically detecting the miRNA marker combination in the kit includes at least one oligonucleotide, wherein at least a part of the oligonucleotide specifically binds to the miRNA marker in the above-mentioned peripheral blood miRNA marker combination for diagnosing gastric cancer.

In some embodiments, the reagent for specifically detecting the miRNA marker combination in the kit according to the present invention is a detection reagent for qPCR method.

Further, in some embodiments, the detection reagent for qPCR method in the kit according to the present invention includes a reverse transcription primer and/or a qPCR amplification primer of the miRNA marker combination.

Further, in some embodiments, the kit includes a stem-loop reverse transcription primer and/or a semi-nested qPCR primer for amplifying the miRNA markers in the above-mentioned peripheral blood miRNA marker combination for diagnosing gastric cancer.

In some embodiments, the detection reagent for qPCR method in the kit according to the present invention includes a reverse transcription primer and/or a qPCR amplification primer of each miRNA in the miRNA marker combination consisting of twelve miRNA markers: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of eleven miRNA markers: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of ten miRNA markers: hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of nine miRNA markers: hsa-miR-424-5p, hsa-miR-103a-3p, hsa-miR-181a-5p, hsa-miR-21-5p, hsa-miR-140-5p, hsa-miR-30e-5p, hsa-miR-126-3p, hsa-miR-183-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of eight miRNA markers: hsa-miR-424-5p, hsa-miR-103a-3p, hsa-miR-181a-5p, hsa-miR-21-5p, hsa-miR-140-5p, hsa-miR-30e-5p, hsa-miR-126-3p and hsa-miR-340-5p.

In some embodiments, the detection reagent for qPCR method in the kit according to the present invention includes a reverse transcription primer and/or a qPCR amplification primer of each miRNA in the miRNA marker combination consisting of seven miRNA markers: hsa-miR-424-5p, hsa-miR-103a-3p, hsa-miR-181a-5p, hsa-miR-21-5p, hsa-miR-140-5p, hsa-miR-30e-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of six miRNA markers: hsa-miR-424-5p, hsa-miR-103a-3p, hsa-miR-181a-5p, hsa-miR-21-5p, hsa-miR-140-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of five miRNA markers: hsa-miR-103a-3p, hsa-miR-181a-5p, hsa-miR-21-5p, hsa-miR-140-5p and hsa-miR-340-5p.

In some embodiments, the detection reagent for qPCR method in the kit according to the present invention includes a reverse transcription primer and/or a qPCR amplification primer of each miRNA in the miRNA marker combination consisting of four miRNA markers: hsa-miR-103a-3p, hsa-miR-181a-5p, hsa-miR-21-5p and hsa-miR-340-5p.

Further, in some embodiments, the detection reagent for qPCR method in the kit according to the present invention further includes at least one of positive quality control products, negative quality control products, reverse transcriptase, dNTPs, reverse transcription buffer, nuclease-free water , qPCR buffer, magnesium chloride, DNA polymerase, and SYBR Green fluorescent dye.

The present invention also provides a use of the miRNA marker combination in the preparation of a gastric cancer diagnostic reagent for predicting the likelihood that the subject will develop or have gastric cancer by the following method, the method including:

detecting the presence of miRNA in a peripheral blood sample obtained from the subject;
measuring the expression level of at least one miRNA in the miRNA marker combination in the peripheral blood sample; and
predicting the likelihood that the subject will develop or have gastric cancer using the score based on the previously measured expression level of miRNA.

The peripheral blood is serum or plasma.

The expression level of the miRNA is scored by constructing a simple linear regression model using a linear regression algorithm. A linear regression model is used to calculate the risk score. Logistic regression is an example of a linear regression method that can be used for this purpose. However, any relevant person skilled in the art will understand that other forms of linear regression calculation can be used to calculate and obtain the score. The critical value of the risk score is determined based on clinical needs, and two critical values are used to define the population as a high-risk population, a low-risk population, and an uncertain population.

The subjects include, but are not limited to, Chinese, Malaysian, and Indian.

It can be seen from the above technical solutions that the present invention provides a marker combination and a kit for diagnosing gastric cancer. The combination of miRNA markers for diagnosing gastric cancer according to the present invention includes at least four miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p. The detection of the serum in a subject using the combination of markers for diagnosing gastric cancer can distinguish serum of a patient with gastric cancer from that of a Healthy human, and distinguish serum of a patient with gastric cancer from that of a patient with gastritis. The above-mentioned miRNA marker combination according the present invention is used to test the subject. The results are compared with the Helicobacter pylori test and the pepsinogen test, which shows good agreement with the clinical gold standard of endoscopy, and is significantly better than the two existing biomarkers of pepsinogen I/II ratio and Helicobacter pylori test respectively. The kit for diagnosing gastric cancer according to the present invention has a simple composition, and can detect gastric cancer simply, effectively and non-invasively.

**Brief Description of Figures**

In order to explain the embodiments of the present invention or the technical solutions in the prior art more clearly, the following will briefly introduce the drawings that need to be used in the description of the embodiments or the prior art.

Figure 1 shows the roadmap for clinical validation of serum miRNA in gastric cancer;

Figure 2 is a diagram showing the ROC characteristics of different blood markers;

Figure 3 is a diagram showing the ROC characteristics of male and female;

Figure 4 is a diagram showing the ROC characteristics of subjects of different races in this kit;

Figure 5 is a diagram showing the ROC characteristics of subjects with early gastric cancer and late gastric cancer in this kit;

Figure 6 is a diagram showing the ROC characteristics of combinations with different miRNA numbers under linear regression algorithm.

**Detailed Description**

The invention discloses a miRNA marker combination and a kit for diagnosing gastric cancer. Those skilled in the art can learn from the content of this article and appropriately improve the process parameters to achieve the present invention. In particular, it should be pointed out that all similar substitutions and modifications are obvious to those skilled in the art, and they are all deemed to be included in the present invention. The method and product of the present invention have been described through the preferred embodiments. It is obvious that relevant persons can make changes or appropriate changes and combinations to the methods described herein without departing from the content, spirit and scope of the present invention to realize and apply the technology of the present invention.

In order to further understand the present invention, the technical solutions in the examples of the present invention will be clearly and completely described below in conjunction with the examples of the present invention. Obviously, the described examples are only a part of the examples of the present invention, rather than all the examples. Based on the examples of the present invention, all other examples obtained by those of ordinary skill in the art without creative work shall fall within the protection scope of the present invention.

Unless otherwise specified, the reagents involved in the examples of the present invention are all commercially available products, and all of which can be purchased through commercial channels. The design method of the reverse transcription primers and RT-qPCR primers of the miRNA according to the present invention is performed in accordance with the methods as described in US Patent Publication No. US9850527B2 and Wan G, Lim Q', Too H P. High-performance quantification of mature microRNAs by real-time RT -PCR using deoxyuridine-incorporated oligonucleotides and hemi-nested primers [J]. Rna-a Publication of the Rna Society, 2010, 16(7):1436-45. All miRNA sequences disclosed in the present invention have been stored in the miRBase database (http://www.mirbase.org/).

Example 1

In the development phase, RT-qPCR technology was used to detect the serum miRNA of 236 gastric cancer patients and 236 non-cancer control subjects. 191 miRNAs (expression level ≥500 copies/ml) can be detected in more than 90% of subjects; 75 of the 191 miRNAs were differentially expressed between the control group and cancer patients (FDR P value<0.01). Among the 75 differentially expressed miRNAs, 51 were up-regulated in gastric cancer patients and 24 were down-regulated.

In the validation phase, RT-qPCR technology was used to detect the serum miRNA of 94 gastric cancer patients and 116 non-cancer control subjects. There was a good correlation between the fold change of miRNA expression for the development phase and the validation phase. Finally, 12 miRNAs were further selected as biomarkers and combinations for gastric cancer detection, specifically hsa-miR-29c-3p, hsa-miR-424-5p, hsa-miR-103a-3p, hsa-miR-93-5p , hsa-miR-181a-5p, hsa-miR-21-5p, hsa-miR-140-5p, hsa-miR-30e-5p, hsa-miR-142-5p, hsa-miR-126-3p, hsa-miR-183-5p, and hsa-miR-340-5p. Each of the miRNA sequences and miRBase database registration numbers is shown in Table 1.

Table 1 12 miRNA sequences and miRBase database registration numbers

| miRNA | Registration number | Sequence (5'→3') |
|---|---|---|
| hsa-miR-29c-3p | MIMAT0000681 | UAGCACCAUUUGAAAUCGGUUA |
| hsa-miR-424-5p | MIMAT0001341 | CAGCAGCAAUUCAUGUUUUGAA |
| hsa-miR-103a-3p | MIMAT0000101 | AGCAGCAUUGUACAGGGCUAUGA |
| hsa-miR-93-5p | MIMAT0000093 | CAAAGUGCUGUUCGUGCAGGUAG |
| hsa-miR-181a-5p | MIMAT0000256 | AACAUUCAACGCUGUCGGUGAGU |
| hsa-miR-21-5p | MIMAT0000076 | UAGCUUAUCAGACUGAUGUUGA |
| hsa-miR-140-5p | MIMAT0000431 | CAGUGGUUUUACCCUAUGGUAG |

(continued)

| miRNA | Registration number | Sequence (5'→3') |
|---|---|---|
| hsa-miR-30e-5p | MIMAT0000692 | UGUAAACAUCCUUGACUGGAAG |
| hsa-miR-142-5p | MIMAT0000433 | CAUAAAGUAGAAAGCACUACU |
| hsa-miR-126-3p | MIMAT0000445 | UCGUACCGUGAGUAAUAAUGCG |
| hsa-miR-183-5p | MIMAT0000261 | UAUGGCACUGGUAGAAUUCACU |
| hsa-miR-340-5p | MIMAT0004692 | UUAUAAAGCAAUGAGACUGAUU |

Two hundred times of two-fold cross-validation was performed on the combinations of 12 miRNA biomarkers obtained from the screening and non-parametric factors to test the performance of the biomarker combination of 2 to 12 miRNAs. AUC was used as an optimization index to construct a simple linear regression model. A linear regression model was used to calculate the risk score. In this example, a logistic regression model was used. The critical value of the risk score was determined based on clinical needs, and two critical values were used to define the population as a high-risk population, a low-risk population, and an uncertain population. The Logistic score of gastric cancer population was significantly higher than that of healthy population.

The calculation formula for the linear regression model is as follows:

$$
\begin{aligned}
\text{Logistic} = & (K_{hsa\text{-}miR29c\text{-}3p} * Ct_{hsa\text{-}miR\ 29c\text{-}3p}) + (K_{hsa\text{-}miR\ 424\text{-}5p} * Ct_{hsa\text{-}miR\ 424\text{-}5p}) \\
& + (K_{hsa\text{-}miR\ 103a\text{-}3p} * Ct_{hsa\text{-}miR\ 103a\text{-}3p}) + (K_{hsa\text{-}miR\ 93\text{-}5p} * Ct_{hsa\text{-}miR\ 93\text{-}5p}) + (K_{hsa\text{-}miR\ 181a\text{-}5p} * Ct_{hsa\text{-}miR\ 181a\text{-}5p}) \\
& + (K_{hsa\text{-}miR\ 21\text{-}5p} * Ct_{hsa\text{-}miR\ 21\text{-}5p}) + (K_{hsa\text{-}miR\ 140\text{-}5p} * Ct_{hsa\text{-}miR\ 140\text{-}5p}) \\
& + (K_{hsa\text{-}miR\ 30e\text{-}5p} * Ct_{hsa\text{-}miR\ 30e\text{-}5p}) + (K_{hsa\text{-}miR\ 142\text{-}5p} * Ct_{hsa\text{-}miR\ 142\text{-}5p}) \\
& + (K_{hsa\text{-}miR\ 126\text{-}3p} * Ct_{hsa\text{-}miR\ 126\text{-}3p}) + (K_{hsa\text{-}miR\ 183\text{-}5p} * Ct_{hsa\text{-}miR\ 183\text{-}5p}) \\
& + (K_{hsa\text{-}miR\ 340\text{-}5p} * Ct_{hsa\text{-}miR\ 340\text{-}5p}) + \text{Constant}
\end{aligned}
$$

wherein, K is the coefficient of each miRNA marker, and the coefficient of each marker is different for different combinations of markers, in which a possible implementation is shown in Table 2.

CT is the relative expression level of each miRNA marker, that is, the Ct value obtained by qPCR.

Table 2 Coefficients of each miRNA marker in different combinations of markers

| miRNA | 12 miRNAs | 11 miRNAs | 10 miRNAs | 9 miRNAs | 8 miRNAs | 7 miRNAs | 6 miRNAs | 5 miRNAs | 4 miRNAs |
|---|---|---|---|---|---|---|---|---|---|
| hsa-miR-29c-3p | -4.16 | -4.82 | | | | | | | |
| hsa-miR-424-5p | -9.31 | -8.40 | -9.09 | -7.93 | -7.48 | -6.74 | -7,55 | | |
| hsa-miR-103a-3p | 15.03 | 15.89 | 15,98 | 14.96 | 6.93 | 5,71 | 4,07 | 1.81 | -5.28 |
| hsa-miR-93-5p | -4.46 | -3.88 | -4.27 | | | | | | |
| hsa-miR-181a-5p | 19.04 | 15.22 | 14.57 | 15.14 | 19.91 | 19.47 | 20.07 | 21.63 | 27.02 |
| hsa-miR-21-5p | -21.16 | -15.55 | -16.22 | -14.58 | -13.61 | -13.74 | -13.26 | -14.24 | -25.59 |
| hsa-miR-140-5p | -12.25 | -14.29 | -14.83 | -16.91 | -20,40 | -20.39 | -20.36 | -25.82 | |
| hsa-miR-30e-5p | 7.63 | 10,75 | 8.44 | 4.85 | -3.24 | -2.18 | | | |
| hsa-miR-142-5p | 2.42 | | | | | | | | |
| hsa-miR-126-3p | -5.96 | -6.86 | -5,85 | -5.88 | -5.45 | | | | |
| hsa-miR-183-5p | -4.89 | -6.10 | -6,41 | -7.35 | | | | | |
| hsa-miR-340-5p | 16.17 | 15.89 | 16.10 | 15.91 | 21.60 | 17.11 | 16.18 | 17.49 | 7.26 |
| Constant | 87.5 | 124.8 | 113.8 | 128.2 | 55.3 | 53.0 | 56,9 | 9.8 | -152.0 |

Example 2

1) Preparation of test samples: collection of blood and separation of serum;

A total of 20ml fasting blood samples were collected in two common serum test tubes. The serum test tubes were centrifuged at 3000 rpm for 10 minutes at 20°C. Centrifugation and serum collection were performed within 4 hours after blood collection. Serum samples were immediately stored at -80°C.

2) RNA extraction;

Total RNA was isolated from 200 $\mu$L of serum using miRNeasy serum/plasma miRNA extraction kit (Qiagen GmbH, Germany). Before RNA extraction, a set of 3 artificially synthesized miRNA controls (incorporated at high, medium and low concentrations) were added to the sample lysis buffer to monitor and standardize the technical differences in this process.

3) Reverse transcription real-time quantitative PCR (RT-qPCR)

Reverse transcription buffer, reverse transcriptase and reverse transcription primers were used to the reverse transcription of miRNA under specific reaction conditions and temperature.

The reverse transcription product (cDNA) was amplified by qPCR using qPCR buffer, DNA polymerase and qPCR plate containing miRNA sequence-specific primers under specific reaction conditions and temperatures. The Ct value of each miRNA was obtained by setting a threshold.

Before RT-qPCR, a set of artificially synthesized miRNA (3) controls was added to each sample to monitor and standardize the technical differences in this process. During the RT-qPCR, each miRNA was added with 6 logarithmically diluted and artificially synthetic templates, negative controls and mixed human serum RNA reference materials, and each isolated serum RNA sample was reverse-transcribed and quantified by qPCR. These quality control measures helped to monitor and standardize technical differences in pipetting and measurement efficiency during RT, cDNA amplification, and qPCR.

4) Protocol for detecting 12-miRNA in the validation phase (Example 3)

The serum sample was lysed by phenol/guanidine, and the total RNA of the serum sample was separated by a silica purification column. In the reverse transcription step, the corresponding stem-loop primers for miRNA reverse transcription were used to reverse-transcribe 12 miRNAs in each sample into cDNA. Subsequently, qPCR was performed using sequence-specific forward PCR primers and semi-nested sequence-specific reverse PCR primers, and SYBR Green I dye was used for detection.

5) The Ct value was imported into the linear regression model described in Example 1 to perform statistical analysis and thus obtain the risk value for suffering from gastric cancer.

Example 3

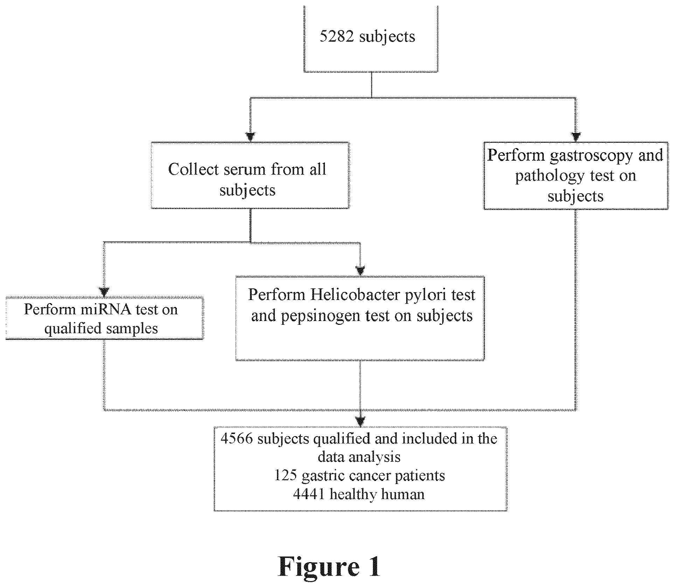

5282 subjects from the National University Hospital in Singapore and Tan Tock Seng Hospital in Singapore were selected for clinical testing. The specific validation route is shown in Figure 1. A total of 5282 subjects entered the clinical validation. According to the method described in Example 2, the 12 miRNA biomarkers described in Example 1 were selected for miRNA test, Helicobacter pylori test and pepsinogen test, as well as gastroscopy and pathology test for all subjects. The 12-miR qPCR assay was developed and manufactured according to the ISO13485 medical equipment quality management system. In the absence of endoscopic and histopathological test results, miRNA testing was performed in a CAP/ISO certified laboratory. Western blot assay was used to determine the Helicobacter pylori antibodies in the serum samples. The levels of pepsinogen I and II were determined with a latex agglutination turbidimetric immunoassay kit. Both determinations were performed when the clinical results were unknown. After screening, 4566 subjects were finally used for data analysis, 125 subjects were diagnosed with gastric cancer, and 4441 subjects were confirmed as Healthy human without cancer. Table 3 shows the analysis results of the clinical characteristics data of 4566 subjects.

Table 3 Analysis of clinical characteristics data of 4566 subjects

| Parameter statistics | All subjects | Healthy human | Cancer patient |
|---|---|---|---|
| Age (years) | | | |

(continued)

| Parameter statistics | All subjects | | Healthy human | | Cancer patient | |
|---|---|---|---|---|---|---|
| Mean (standard deviation) | 57.17 | ( 10.48 ) | 57.17 | ( 10.48 ) | 56.9 | ( 10.31 ) |
| Median | 56 | | 56 | | 56 | |
| Minimum | 38 | | 38 | | 39 | |
| Maximum | 94 | | 94 | | 94 | |
| Gender | | | | | | |
| Male (%) | 2422 | ( 53.04% ) | 2346 | ( 52.83% ) | 76 | ( 60.80% ) |
| Female (%) | 2144 | ( 46.96% ) | 2095 | ( 47. 17% ) | 49 | ( 39.20% ) |
| Races | | | | | | |
| Chinese (%) | 3490 | ( 76.43% ) | 3394 | ( 76.42% ) | 96 | ( 76.80% ) |
| Malaysian (%) | 332 | ( 7.27%. ) | 325 | ( 7.32% ) | 7 | ( 5.60% ) |
| Indian (%) | 378 | ( 8.28% ) | 369 | ( 8,31%) | 9 | ( 7,20% ) |
| Others (%) | 366 | ( 8.02% ) | 353 | ( 7.95% ) | 13 | ( 10.40% ) |
| Cancer staging (AJCC 2010) | | | | | | |
| Stage 0 (%) | - | | - | | 10 | ( 8.00% ) |
| Stage I (%) | - | | - | | 16 | ( 12,80% ) |
| Stage II (%) | - | | - | | 20 | ( 16 00% ) |
| Stage III (%) | - | | - | | 31 | ( 24.80% ) |
| Stage IV (%) | - | | - | | 38 | ( 30.40% ) |
| Unknown (%) | - | | - | | 10 | ( 8.00% ) |
| Pepsinogen I/II | | | | | | |
| Mean (standard deviation) | 11.23 | ( 31.11 ) | 11,27 | ( 31.11 ) | 9.6 | ( 31,16 ) |
| Median | 6 | | 6 | | 5.3 | |
| Minimum | 0.2 | | 0.2 | | 0,4 | |
| Maximum | 594.1 | | 594,1 | | 280 | |
| Helicobacter pylori | | | | | | |
| Positive (%) | 2530 | ( 55.41% ) | 2426 | ( 54.63% ) | 104 | ( 83.20% ) |
| Negtive(%) | 2036 | ( 44,59% ) | 2015 | ( 45.37% ) | 21 | ( 16,80% ) |

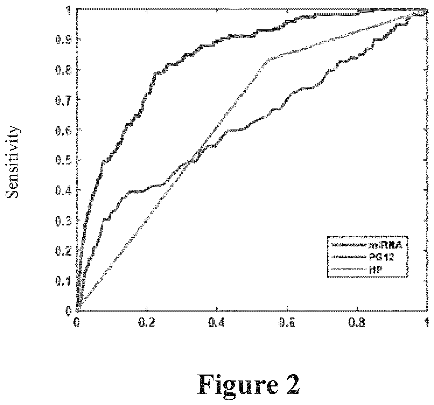

The miRNA marker combination test method according to the present invention, Helicobacter pylori test and pepsinogen test were compared, and the results are shown in Figure 2 and Table 4.

Table 4 AUC results of different blood markers

| Blood markers | AUC |
|---|---|
| The miRNA marker combination test according to the present invention | 0.84 |
| PG12-pepsinogen I/II ratio | J.62 |
| HP-Helicobacter pylori | J.64 |

It can be seen from the results of the 4566 subjects that the miRNA marker combination test method according to the present invention showed good agreement with the clinical gold standard of endoscopy (AUC is 0.84), and was significantly better than the two existing biomarkers of pepsinogen I/II ratio and Helicobacter pylori test respectively (AUC is 0.62 and 0.64). The AUC (0.84) of 4566 subjects was close to the AUC (0.89) of the algorithm development cohort, which proved the consistency of the experimental results between the development cohort for the miRNA marker combination according to the present invention and the clinical validation cohort for the blind test.

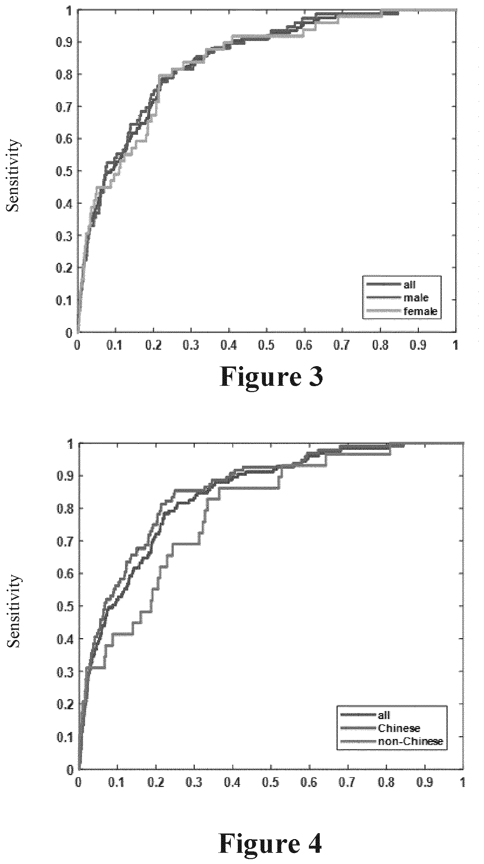

The gender differences in clinical validation data were further analyzed. The miRNA marker combination test according to the present invention showed consistent AUC in male and female subjects (Figure 3 and Table 5).

Table 5 AUC results of male and female subjects

| Blood markers | AUC |
| --- | --- |
| All subjects | 0.84 |
| Male subjects | 0.85 |
| Female subjects | 0.84 |

The clinical validation data was further analyzed to determine whether the performance of the miRNA marker combination test according to the present invention on populations of different races is different or not. Generally consistent with Singapore's demographic data, most of the subjects participating in this clinical trial are Chinese (76.43%), and each of Malaysian, Indian and other ethnic groups accounted for about 8% (Table 3). The results showed that the AUC is Chinese subjects was higher than that of other races (Figure 4, Table 6).

Table 6 AUC results of subjects of different races

| Blood markers | AUC |
| --- | --- |
| All subjects | 0.84 |
| Chinese subjects | 0.86 |
| Subjects of other races | 0.79 |

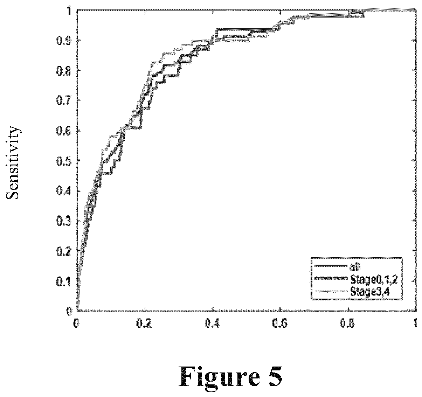

The performance of the miRNA marker combination test according to the present invention was further evaluated by cancer staging. The miRNA marker combination test according to the present invention showed that the AUC of early (stage 0, I and II) and advanced (stage III and IV) cancers were 0.83 and 0.85, respectively (Figure 5, Table 7).

Table 7 AUC results of subjects with early gastric cancer and advanced gastric cancer

| Blood markers | AUC |
| --- | --- |
| All subjects | 0.84 |
| Subjects with early gastric cancer | 0.83 |
| Subjects with advanced gastric cancer | 0.85 |

In addition, the clinical specificity of miRNA detection for 7 other common cancers including lung cancer, breast cancer, colorectal cancer, liver cancer, esophageal cancer, prostate cancer and bladder cancer had been demonstrated. This assay had very little cross-reactivity with other common cancers including gastrointestinal cancers (Table 8).

Table 8 Cross-reactivity test with other common cancers

| # | Cancer type | Number of test samples | Number of specimens with high risk score |
| --- | --- | --- | --- |
| 1 | Esophageal Cancer | 12 | 1 |
| 2 | Liver Cancer | 6 | 1 |
| 3 | Colorectal Cancer | 12 | 3 |
| 4 | Lung Cancer | 12 | 1 |
| 5 | Breast Cancer | 12 | 0 |
| 6 | Prostate Cancer | 12 | 0 |
| 7 | Bladder Cancer | 12 | 0 |
| | Total | 78 | 6 |

Example 4

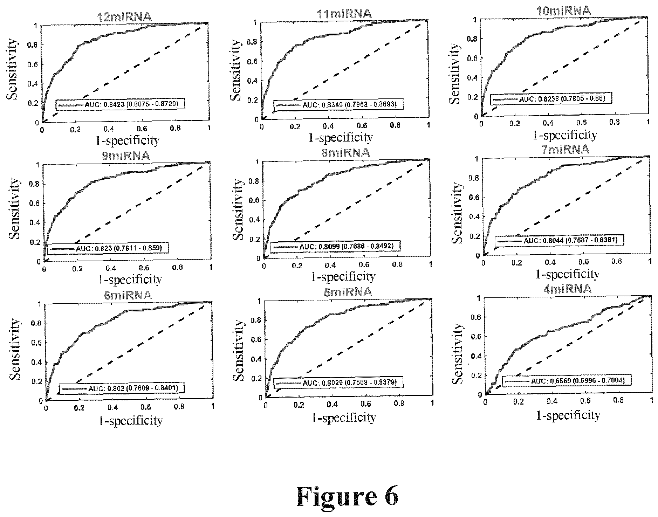

According to the method described in Example 2, the combinations of 12, 11, 10, 9, 8, 7, 6, 5, and 4 miRNAs described in Example 1 were sequentially selected in accordance with the important order of miRNA and tested using the cancer and control subject samples described in Example 3 to obtain the diagrams showing the ROC characteristics (Figure 6, Table 9).

Table 9 Parameters of the combinations with different miRNA numbers under Logistic algorithm

| | | 12 miRNAS | 11 miRNAs | 10 miRNAs | 9 miRNAs | 8 miRNAs | 7 miRNAs | 6 miRNAs | 5 miRNAs | 4 miRNAs |
|---|---|---|---|---|---|---|---|---|---|---|
| 40 cutoff | Sensitivity | 84.8% (83.7% - 85.8%) | 84.0% (82.9% - 85.0%) | 83.2% (82.1%- 84.3%) | 81.6% 80.4%- 82.7%) | 80.0% 78.8% - 81.1%) | 79.2% (78.0% 80.4%) | 79,2% (78.0%- 80.4%) | 77.6% (76.4% 78.8%) | 58,4% -(57.0%- 59.8%) |
| | Specificity | 68.4% (67.1% - 69.8%) | (66.2% - 69.0%) | 67.4% (66.0% - 68.8%) | 67.6% (66.2% - 69.0%) | 68.4% (67.0%- 69.7%) | 69.0% (67.7% 70.4%) | 68.8% -(67.4% - 70.1%) | 68.2% (66.9%- 69.6%) | 68.2% (66.9% - 69.6%) |
| | Accuracy | 68.9% (67.5%- 70.2%) | 68.0% 66.7% - 69.4%) | 67.8% (66., 4%,-69.2%) | 68.0% -(66,6% - 69.3%) | 68.7% (67.3% - 70.0%) | | 69.1% (67.7%- 70.4%) | 68.5% (67.1% - 59.8%) | if>8.0% -(66.6%- 69.3%) · |
| | PPV | 7.0% (6.3% - 7.8%) | 6.8% (6.1% - 7.6%) | 6.7% 7.5%) | 6.6% (5.9%- 7.4%) | 6.6% 6.0% - 7.4%) | 6.7% 6.0% - 7.5%) | 6.7% 6.0% - 7.4%) | 6.4% (5.7% - 7.2%) | 4.9% (4.3% - 5.6%) |
| | NPV | 99.4% (99.1% 99.6%) | 99.3% (99.0% - 99.5%) | 99.3 % (99.0)%-99.5%) | 99.2% -(98.9% - 99.5%) | 99.2% (98.9% - | 99.2% (98.8% - 99.4%) | 99.2% (98.8% - 99,4%) | 99.1% (98.8% - 199.3%) | 98.3% (97.9% - 98,7%) |
| | AUC | 0.842 (0,809- 0.875) | 0.835 (0,708 - 0.871) | 0.834 (0.797 - 0.871) | 0.833 (0,796 - 0.870) | 0.815 (0.779- 0.852) | 0.808 (0.770 - 0.844) | 0.807 (0.770 - 0.843) | 0.803 (0,764 - 0.841) | 0.657 (0.605 - 0.709) |

(continued)

| | | 12 miRNA | 11 miRNA | 10 miRNA | 9 miRNA | 8 miRNA | 7 miRNA | 6 miRNA | 5 miRNA | 4 miRNA |
|---|---|---|---|---|---|---|---|---|---|---|
| 50 cut-$^1_3$11 | Sensitivity | 44,0% (42.6% - 45.5%) | 44,0% (42.6% - 45.5%) | 45,6% (44.1% - 47.1%) | 47.2% (45.7% - 48.7%) | 43,2% (41.8% - 44.7%) | 40.8% (39.4% - 42.2%) | 40,0%, -. (38.6% - 41.4%) | 37.6% (36.2% - 39.0%) | 20,8% -j() 9.6% - 22.0%) |
| | Specificity | 93.9% (93.2% - 94,6%) | 93.6% (92.8% - 94.2%) | 93.4% 92.7% - <92.7% - 94.1%) | 93 .4% (92,6% - 94.1%) | 92. 7% -(91.9%- 93.4%) | M.3%(91.5% - 93.0%) | 92.4% (91.5% - 93.1%) | '92 7% (91.9% - 93.4%) | 92,0% (91.2% - 92.8%) |
| | Accuracy | 92.6% (91.8% - 93.3%) | 92.2% (91.4% - 93.0%) | 92,1% (91.3% - 92.9%) | 92.1% - 92.9%) | 91.3% 90.5% - 92.1%) | 90,9% (90.0% - 91.7%) | 90.9% -(90.1% | 91.2% (90.3% - 92.0%) | (89.1% - |
| | PPV | 17.0% (15.9% - 18.1%) | 16.1% (15.1% - 17.2%) | 16.4% 15.3% - 17.5%) | 16.7% (15.6% - 17.8%) | 14.3% (13.3% - 15.3%) | 13.0% (12.0% - 14.0%) | 12.9% (11.9% - (11.9% - 13.9%) | 12.6% (11.7% - 13.6%) | 6 .8% (6.1% - 7.6%) |
| | NPV | 98.3% (97.9% - 98.7%) | 98.3% (97.9% - 98.7%) | 98.4% (98.0% - 98.7%) | 98.4% (98.0% - 98.8%) | 98.3% (97.9% - 98.7%) | 98.2% (97.8% - 98.6%) | 98.2% (97.8% - 98.6%) | 98.1% (97.7% - 98.5%) | 97,6% - (97.1% - 98.0%) |
| | AU C | 0.842 (0.809 - 0.875) | 0.835 (0.798 - 0.871) | 0.834 (0.797 - 0.871) | 0.833 (0.796 - 0.870) | 0.815 (0.779 - 0.852) | 0.808 (0.771 - 1.844) | 0.807 (0.770 - 0.843) | 0.803 (0.764 - 0.841) | Q.657 | (0.605 - 0.709) |

It should be noted that the Logistic algorithm showed different diagnostic performances and was suitable for different diagnostic approaches when different scores were used as the dividing line between cancer and non-cancer (Table 8). For example, when a dividing line of 40 points was used, that is, a patient with a score of 40 or more was defined as a cancer patient, the sensitivity would be significantly higher than the dividing line of 50 points, and the specificity and accuracy would be significantly lower than the dividing line of 50 points. Therefore, the lower Logistic dividing line was suitable for gastric cancer screening in the population, and the higher Logistic dividing line was suitable for auxiliary diagnosis.

Discussion

Although there have been many documents proving that miRNA can be used as a marker for diagnosing cancer and other diseases, there is no consensus on specific miRNA expression profiles for specific diseases. The use of miRNAs as diagnostic or prognostic markers may face certain challenges (Tiberio *et al,* 2015). Even for the same disease, the miRNA markers screened in different studies may be very different (Leidner *et al,* 2013). Due to the lack of consistency in the selection of research subjects, sample collection, processing steps, and detection methods in the existing literature may result in the suboptimal choice of miRNA biomarkers for diagnosis or prognosis. Also, most of the research results in these documents have not been verified in larger research cohorts.

The health and treatment conditions of the subject as well as environmental and genetic factors may all have an impact on the miRNA expression profile. When miRNA analysis is based only on a single study cohort or samples from the same location, it may cause bias in the results. Therefore, it is necessary to take samples from a large enough population and design a well-designed and controllable data analysis workflow to screen miRNA markers that can be applied to large-scale populations. The present invention has fully validated the currently selected 12 miRNA biomarkers, and these validations are performed on different patient cohorts from different research locations. Starting from the initial development phase, miRNA biomarkers are screened and verified in different cohorts. The selected 12 miRNAs are subsequently further validated in a larger prospective clinical study involving 5282 subjects. The 12 miRNAs screened and validated in a large number of subjects from multiple independent research cohorts ensures their robustness as a biomarker and can be applied to a wide range of populations to identify subjects at risk of gastric cancer.

The miRNA profile may also be affected in the aspect of technologies, such as sample collection, processing steps, and methods for detecting miRNA expression level in the sample. The sensitivity and specificity for detecting changes in miRNA expression may vary significantly due to different detection methods. In most published studies, sample collection and processing methods can be quite different. In addition, there are differences in the reagents and methods used in the studies. A semi-nested primer is designed herein based on the principle of Wan *et al,* 2010, adopts a highly sensitive and specific RT-qPCR method and an optimized miRNA expression profile and data analysis workflow, and the internal references are used to monitor and control efficiency differences in miRNA extraction, reverse transcription, qPCR and other steps. In addition, the clinical validation study of 5282 subjects adopts a more demanding method than the clinical validation of medical diagnostic tests (Wilson *et al,* 2008; Mattocks *et al,* 2010). The test kit is produced and executed under strict quality control requirements, which is not common in clinical research. The technical method according to the present invention further ensures the effectiveness and robustness of the 12 miRNA markers used to identify the risk of gastric cancer.

In a prospective study for validating 12 miRNA biomarkers that 5282 subjects participated in, these subjects are also tested for other risk factors relating to gastric cancer, such as Helicobacter pylori, pepsinogen I/II test, and gastroscopy. The results show that the detection of 12-miRNA combination is significantly better than some conventional clinical tests for high-risk factors of gastric cancer, such as Helicobacter pylori and pepsinogen I/II in terms of identifying patients with gastric cancer. The 4-miRNA group has a closer AUC, compared with pepsinogen 1/2 test (AUC=0.62) and Helicobacter pylori (AUC=0.64). The miRNA combinations having 5 or more miRNAs have a better AUC for the diagnosis of gastric cancer (AUC of 5-miRNA group is 0.8029, and AUC of 12-miRNA group is 0.8423). The AUC of miRNA detection composed of 5 or more miRNAs is very close to that of gastroscopy (AUC is 0.84), which is currently the gold standard for gastric cancer diagnosis.

Subjects who are determined to be at risk of gastric cancer may need further tests, for example gastroscopy, biopsy, or diagnostic imaging tests, such as magnetic resonance imaging (MRI) or computed tomography (CT). Patients diagnosed with gastric cancer will receive appropriate treatment. The treatment of gastric cancer generally includes one or more of the following interventions: surgery, radiotherapy, chemotherapy or immunotherapy, or the use of targeted therapies such as trastuzumab and ramucirumab. Potential drugs for gastric cancer treatment include small molecules, antibodies, vaccines, or peptides. Chemotherapeutic drugs for gastric cancer treatment include 5-fluorouracil, capecitabine, carboplatin, cisplatin, docetaxel, epirubicin, irinotecan, oxaliplatin, paclitaxel, trifluridine, and tipiracil. Immunotherapeutic drugs for gastric cancer treatment include immune checkpoint inhibitors, such as pembrolizumab. The treatment option for early gastric cancer is usually surgery. For cancers that are detected early, endoscopic resection is also possible. It is generally believed that the therapeutic effect in patients with early gastric cancer is significantly better than that in patients with advanced gastric cancer (Lello *et al,* 2007). Therefore, a reliable and easy-to-use detection method is of great significance for the early diagnosis of gastric cancer patients.

Therefore, the present invention provides in detail the validation data of a large-scale clinical study for the combinations with 12 miRNA biomarkers. Compared with conventional detection methods for assessing the risk of gastric cancer, the miRNA marker combination can provide an AUC equivalent to gastroscopy for the diagnosis of gastric cancer, but it does not have the invasiveness of gastroscopy and the risk of related complications. A key principle of population cancer screening is to insist on regular screening to facilitate early detection of cancer. In this regard, compared with more invasive tests, a blood-based test that only requires a single blood draw and provides reliable conclusions will be a more promising development direction.

**Claims**

1. A peripheral blood miRNA marker combination for diagnosing gastric cancer, **characterized in that** it includes at least five miRNA markers selected from: hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

2. The miRNA marker combination according to claim 1, **characterized in that** it consists of hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-142-5p, hsa-miR-126-3p, hsa-miR-183-5p and hsa-miR-340-5p.

3. The miRNA marker combination according to claim 1, **characterized in that** it consists of hsa-miR-29c-3p, hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

4. The miRNA marker combination according to claim 1, **characterized in that** it consists of hsa-miR-424-5p, hsa-miR-103a-3p, hsa-miR-93-5p, hsa-miR-181a-5p, hsa-miR-21-5p, hsa-miR-140-5p, hsa-miR-30e-5p, hsa-miR-126-3p, hsa-miR-183-5p and hsa-miR-340-5p.

5. The miRNA marker combination according to claim 1, **characterized in that** it consists of hsa-miR-424-5p, hsa-miR-103a-3p, hsa-miR-181a-5p, hsa-miR-21-5p, hsa-miR-140-5p, hsa-miR-30e-5p, hsa-miR-126-3p, hsa-miR-183-5p and hsa-miR-340-5p.

6. The miRNA marker combination according to claim 1, **characterized in that** it consists of hsa-miR-424-5p, hsa-miR-103a-3p, hsa-miR-181a-5p, hsa-miR-21-5p, hsa-miR-140-5p, hsa-miR-30e-5p, hsa-miR-126-3p and hsa-miR-340-5p.

7. The miRNA marker combination according to claim 1, **characterized in that** it consists of hsa-miR-424-5p, hsa-miR-103a-3p, hsa-miR-181a-5p, hsa-miR-21-5p, hsa-miR-140-5p, hsa-miR-30e-5p and hsa-miR-340-5p.

8. The miRNA marker combination according to claim 1, **characterized in that** it consists of hsa-miR-424-5p, hsa-miR-103a-3p, hsa-miR-181a-5p, hsa-miR-21-5p, hsa-miR-140-5p and hsa-miR-340-5p.

9. The miRNA marker combination according to claim 1, **characterized in that** it consists of hsa-miR-103a-3p, hsa-miR-181a-5p, hsa-miR-21-5p, hsa-miR-140-5p and hsa-miR-340-5p.

10. A method for identifying a subject at risk of suffering from gastric cancer, including:

    a. detecting the expression level of the miRNA marker combination according to claim 1 in the subject's peripheral blood sample,
    b. calculating and correcting the risk score of the subject with the non-cancer control sample as a reference, and
    c. determining the possibility of the subject suffering from gastric cancer based on the risk score.

11. The method according to claim 10, **characterized in that** a linear regression model is used to calculate the risk score.

12. The method according to claim 11, **characterized in that** the linear regression model is calculated as follows:

$$\text{Logistic} = (K_{miRNA1}{}^{*}Ct_{miRNA1}) + (K_{miRNA2}{}^{*}Ct_{miRNA2}) + (K_{miRNA3}{}^{*}Ct_{miRNA3}) + \quad \ldots\ldots + \text{Constant}$$

wherein miRNA1, miRNA2, miRNA3... are selected from the miRNA marker according to claim 1; Ct is the relative expression level of each miRNA detected by qPCR; and K is the coefficient of each miRNA marker.

13. The method according to claim 10, wherein the method comprises a qPCR method, a chip method or a sequencing method for detecting the miRNA marker.

14. A kit for diagnosing gastric cancer, **characterized in that** it includes a reagent for specifically detecting the miRNA marker combination according to claim 1.

15. The kit according to claim 14, **characterized in that** the reagent includes at least one oligonucleotide, wherein at least a part of the oligonucleotide specifically binds to the miRNA marker according to claim 1.

16. The kit according to claim 15, wherein the kit includes a stem-loop reverse transcription primer and/or a semi-nested qPCR primer for amplifying the miRNA marker according to claim 1.

5282 subjects

Collect serum from all
subjects

Perform gastroscopy and
pathology test on
subjects

Perform miRNA test on
qualified samples

Perform Helicobacter pylori test
and pepsinogen test on subjects

4566 subjects qualified and included in the
data analysis
125 gastric cancer patients
4441 healthy human

**Figure 1**

**Figure 2**

Figure 3

Figure 4

Figure 5

# Figure 6

| INTERNATIONAL SEARCH REPORT | International application No. |
|---|---|
| | **PCT/CN2020/084687** |

**A. CLASSIFICATION OF SUBJECT MATTER**

C12Q 1/68(2018.01)i

According to International Patent Classification (IPC) or to both national classification and IPC

**B. FIELDS SEARCHED**

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

database: DWPI, SIPOABS, CNABS, ISI Web of knowledge, EMBASE, NCBI PUBMED, CNKI, MEDLINE, Google Scholar: mir-29, mir-424, mir-103, mir-93, mir-181, mir-21, mir-140, mir-30, mir-142, mir-126, mir-183, mir-340, 胃癌, 标记物, 标志物, 线性回归, 试剂盒, Gastric cancer, marker, linear, regression, kit

**C. DOCUMENTS CONSIDERED TO BE RELEVANT**

| Category* | Citation of document, with indication, where appropriate, of the relevant passages | Relevant to claim No. |
|---|---|---|
| PX | CN 110029169 A (MIRUI LABS PTE LTD.) 19 July 2019 (2019-07-19)<br>entire document | 1-16 |
| X | CN 107109470 A (AGENCY FOR SCIENCE TECHNOLOGY AND RESEARCH; NATIONAL UNIVERSITY OF SINGAPORE; NATIONAL UNIVERSITY HOSPITAL) 29 August 2017 (2017-08-29)<br>claims, description, paragraphs 144-153 | 1-16 |
| A | CN 102892897 A (FUDAN UNIVERSITY) 23 January 2013 (2013-01-23)<br>entire document | 1-16 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 May 2020** | **20 July 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/084687**

| Patent document cited in search report | | | Publication date (day/month/year) | Patent family member(s) | | | Publication date (day/month/year) |
|---|---|---|---|---|---|---|---|
| CN | 110029169 | A | 19 July 2019 | None | | | |
| CN | 107109470 | A | 29 August 2017 | US | 2017233822 | A1 | 17 August 2017 |
| | | | | EP | 3177739 | B1 | 23 October 2019 |
| | | | | WO | 2016022076 | A1 | 11 February 2016 |
| | | | | KR | 20170035932 | A | 31 March 2017 |
| | | | | EP | 3177739 | A4 | 02 May 2018 |
| | | | | JP | 2017525350 | A | 07 September 2017 |
| | | | | EP | 3177739 | A1 | 14 June 2017 |
| | | | | SG | 11201700944 R | A | 30 March 2017 |
| CN | 102892897 | A | 23 January 2013 | CN | 102892897 | B | 03 August 2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910392316

- US 9850527 B2

**Non-patent literature cited in the description**

- **WAN G ; LIM Q ; TOO H P.** High-performance quantification of mature microRNAs by real-time RT-PCR using deoxyuridine-incorporated oligonucleotides and hemi-nested primers. *Rna-a Publication of the Rna Society,* 2010, vol. 16 (7), 1436-45